# EUROPÄISCHE PATENTSCHRIFT

(11) **EP 1 673 124 B1**
(45) Veröffentlichungstag und Bekanntmachung des Hinweises auf die Patenterteilung: **13.02.2008**
(21) Anmeldenummer: 04765066.8
(22) Anmeldetag: 10.09.2004
(51) Int. Cl.: A61M 11/06, B05B 7/00

(54) **INHALATIONSTHERAPIEVORRICHTUNG MIT EINEM DÜSENVERNEBLER**
INHALATION THERAPY DEVICE WITH A NOZZLE ATOMIZER
DISPOSITIF DE TRAITEMENT PAR INHALATION MUNI D'UN ATOMISEUR

(30) Priorität: 16.10.2003 DE 10348237
(43) Veröffentlichungstag der Anmeldung: 28.06.2006
(73) Patentinhaber: PARI GmbH Spezialisten für effektive Inhalation, 82319 Starnberg (DE)
(72) Erfinder: KREUTZMANN, Vera, 82229 Seefeld (DE); KUMMER, Frank, 85764 Oberschleissheim (DE); MORNHINWEG, Markus, 86911 Diessen (DE); ROSENBEIGER, Sven, 23564 Lübeck (DE); SELZER, Titus, 80686 München (DE)
(74) Vertreter: HOFFMANN EITLE
(86) Internationale Anmeldenummer: PCT/EP2004/010140
(87) Internationale Veröffentlichungsnummer: WO 2005/042075

(56) Entgegenhaltungen:
- EP-A- 0 855 224
- DE-B1- 1 147 354
- DE-U- 8 905 364
- US-A- 3 097 645
- US-A- 5 301 663
- US-A- 5 511 538

## Beschreibung

Die Erfindung betrifft eine Inhalationstherapievorrichtung mit einem Aerosolerzeuger, der ein Düsenelement aufweist, die leicht zu reinigen und dabei einfach und sicher in der Handhabung ist.

Inhalationstherapievorrichtungen werden verwendet, um Patienten mit Erkrankungen der Atemwege geeignete Medikamente in Form eines Aerosols zu verabreichen. Durch die Einstellung der Tröpfchengröße mittels einer entsprechenden Auslegung eines Verneblers kann gesteuert werden, an welchen Stellen (Rachenraum, Bronchien, Lungen) das Medikament deponiert werden/soll. Dabei atmet der Patient das vernebelte Medikament durch den Mund über ein Mundstück ein, um eine optimale Adaption der Inhalationstherapievorrichtung an den Patienten zu erreichen.

Für die Erzeugung des Aerosols mit einem gewünschten Tröpfchenspektrum ist es notwendig, die Geometrie des Verneblers bzw. des Aerosolerzeugers präzise zu fertigen, um Abweichungen und Veränderungen über die Lebensdauer der Inhalationstherapievorrichtung zu vermeiden. Eine wesentliche Rolle spielt dabei die Geometrie des Düsenelements, wobei das Düsenelement einen Teil des Aerosolerzeugers darstellt. Durch eine präzise Fertigung des Düsenelements wird somit gewährleistet, dass das Aerosol ein reproduzierbares Tröpfchenspektrum aufweist.

Der Aerosolerzeuger und die Düse sind bei einer Inhalationstherapievorrichtung in der Regel Verschmutzungen durch Medikamentenreste, Sputum (Speichel) und Ausatemkondensat ausgesetzt. Um den hygienischen Anforderungen zu entsprechen, vor allem bei Verwendung der Inhalationstherapievorrichtung bei mehreren Patienten, müssen die Verneblerkomponenten daher regelmäßig gereinigt werden, um sie von Medikamentenresten, Ausatemkondensat und Sputumresten zu befreien. Zu diesem Zweck müssen die Verneblerkomponenten derart beschaffen sein, dass sie auf einfache Weise eine gute Reinigung ermöglichen. Eine Inhalationstherapievorrichtung ist in der Regel dazu ausgelegt, dass sie gesäubert und sterilisiert werden kann, um Medikamentenreste, Sputumreste und oder sonstige Verschmutzungen zu entfernen. Zu diesem Zweck kann die Inhalationstherapievorrichtung in der Regel derart geöffnet beziehungsweise zerlegt werden, dass eine Reinigung beziehungsweise ein Sterilisieren problemlos möglich ist.

Die Düse eines Verneblers bzw. eines Aerosolerzeugers weist oftmals scharfe, präzise hergestellte Kanten auf, die notwendig sind, um ein reproduzierbares Tröpfchenspektrum und eine gute Ausbeute, d.h. Effizienz des Verneblers zu erhalten. Diese Kantengeometrien sind sehr empfindlich, speziell bei der Reinigung der Düse, so dass sie nur mit Mühe und großer Vorsicht gereinigt werden können. Letztlich kann mittel- bis langfristig eine Beschädigung der Düsengeometrie praktisch nicht verhindert werden.

Eine Reinigung der Verneblerkomponenten beziehungsweise der Düse muss jedoch aus den eingangs bereits genannten Gründen ermöglicht werden. Diese Reinigung muss für den Patienten, der die Problematik der empfindlichen Düsengeometrie in der Regel nicht kennt trotzdem problemlos möglich sein, vor allem für Patienten, die im Zusammenhang mit ihrer Atemwegserkrankung körperlich eingeschränkt sind.

Im Stand der Technik sind Inhalationstherapievorrichtungen mit Verneblern bzw. Aeosolerzeugern bekannt, beispielsweise aus EP 0 786 263, EP 0 855 224 und US 3 097 645, die derart zerlegt werden können, dass sie beispielsweise unter fließendem Wasser gereinigt werden können, beziehungsweise in einem Autoklav sterilisiert werden können. Zu diesem Zweck kann die Inhalationstherapievorrichtung derart geöffnet werden, dass die Düse des Aerosolerzeugers frei zugänglich ist, so dass sie von einer Reinigungsflüssigkeit erreicht werden kann. Bei fest anhaftenden Partikeln reicht jedoch im Regelfall das Umspülen mit einer Reinigungsflüssigkeit nicht aus, so dass gegebenenfalls eine mechanische Reinigung der Düse vorgenommen werden muss. Das führt unweigerlich zu der Verwendung eines Reinigungswerkzeuges, beispielsweise einer Bürste oder eines Tuchs. Dadurch wird jedoch die Gefahr erheblich erhöht die empfindliche Kantengeometrie der Düse zu beschädigen, und somit das gewünschte Tröpfchenspektrum des Aerosolerzeugers der Inhalationstherapievorrichtung zu verändern. Die Inhalationstherapievorrichtung mit einem Vernebler bzw. einem Aerosolerzeuger würde in vielen Fällen wirkungslos werden, da das Tröpfchenspektrum wesentlich für die Art der Therapie ist.

EP 0 855 224 offenbart eine Inhalationstherapievorrichtung gemäß dem Oberbegriff des Anspruchs 1, und US 3 097 645 offenbart eine Inhalationstherapievorrichtung gemäß dem Oberbegriff des Anspruchs 10.

Die Aufgabe der vorliegenden Erfindung ist es, die Nachteile der Inhalationstherapievorrichtungen im Stand der Technik zu beseitigen und eine Inhalationstherapievorrichtung bereitzustellen, die einen Aerosolerzeuger mit einer Düse aufweist, die konstruktionsbedingt leicht zu reinigen und dabei in der Handhabung sicher und einfach ist, so dass bei der Reinigung keine Beschädigung der Düse und Beeinträchtigung der Düsengeometrie auftritt.

Diese Aufgabe wird gelöst durch eine Inhalationstherapievorrichtung mit einer Verneblerkammer und einem Aerosolerzeuger, der derart angeordnet ist, dass er ein Aerosol in die Verneblerkammer abgeben kann und der Aerosolerzeuger ein Düsenelement umfasst, wobei die Düse aus wenigstens einem ersten Teil und einem zweiten Teil besteht, wobei der erste Teil des Düsenelements aus einem elastischeren Material besteht als der zweite Teil des Düsenelements, und der erste Teil des Düsenelements an dem zweiten Teil des Düsenelements befestigt ist.

Vorteilhafterweise weist der erste Teil des Düsenelements einen sich weiter verjüngenden Querschnitt auf als der zweite Teil des Düsenelements.

Der erste Teil der Düse besteht vorteilhafterweise aus Silikongummi oder einem theromplastischen Elastomer (TPE). Der erste Teil des Düsenelements ist vorteilhafterweise mit dem zweiten Teil des Düsenelements im 2-K-Verfahren gefertigt, wobei dadurch der erste Teil des Düsenelements an den zweiten Teil des Düsenelements angeformt ist.

Vorteilhafterweise umfasst der erste Teil des Düsenelements die Düsenaustrittsöffnung.

Vorteilhafterweise umfasst die Düse gemäß einer weiteren Ausführungsform einen dritten Teil, der die Düsenaustrittsöffnung umfasst.

Der dritte Teil des Düsenelements weist vorteilhafterweise einen sich weiter verjüngenden Querschnitt auf als der erste Teil des Düsenelements.

Vorzugsweise wird der dritte Teil des Düsenelements mit dem ersten Teil des Düsenelements im 2-K-Verfahren hergestellt.

Der dritte Teil des Düsenelements besteht vorteilhafterweise aus einem weniger elastischeren Material, als der erste Teil des Düsenelements

Die Aufgabe der vorliegenden Erfindung wird ferner gelöst durch eine Inhalationstherapievorrichtung mit einer Verneblerkammer und einem Aerosolerzeuger, der derart angeordnet ist, dass er ein Aerosol in die Verneblerkammer abgeben kann und der Aerosolerzeuger ein Düsenelement umfasst, wobei das Düsenelement aus wenigstens einem ersten Teil besteht, wobei der erste Teil des Düsenelements aus einem elastischeren Material besteht als ein Element der Inhalationstherapievorrichtung, an den das Düsenelement angeformt ist, beziehungsweise an dem das Düsenelement befestigt ist.

Im Folgenden wird die Erfindung anhand von Ausführungsbeispielen unter Bezugnahme auf die Zeichnungen genauer beschrieben. In den Zeichnungen zeigt:
- Figur 1: eine Inhalationstherapievorrichtung gemäß einer ersten Ausführungsform der vorliegenden Erfindung;
- Figur 2: das Düsenelement des Aerosolerzeugers der Inhalationstherapievorrichtung gemäß der ersten Ausführungsform der vorliegenden Erfindung;
- Figur 3: ein Düsenelement eines Aerosolerzeugers einer Inhalationstherapievorrichtung gemäß einer zweiten Ausführungsform der vorliegenden Erfindung;
- Figur 4: ein Düsenelement eines Aerosolerzeugers einer Inhalationstherapievorrichtung gemäß einer dritten Ausführungsform der vorliegenden Erfindung; und
- Figur 5: ein Düsenelement eines Aerosolerzeugers einer Inhalationstherapievorrichtung gemäß einer vierten Ausführungsform der vorliegenden Erfindung.

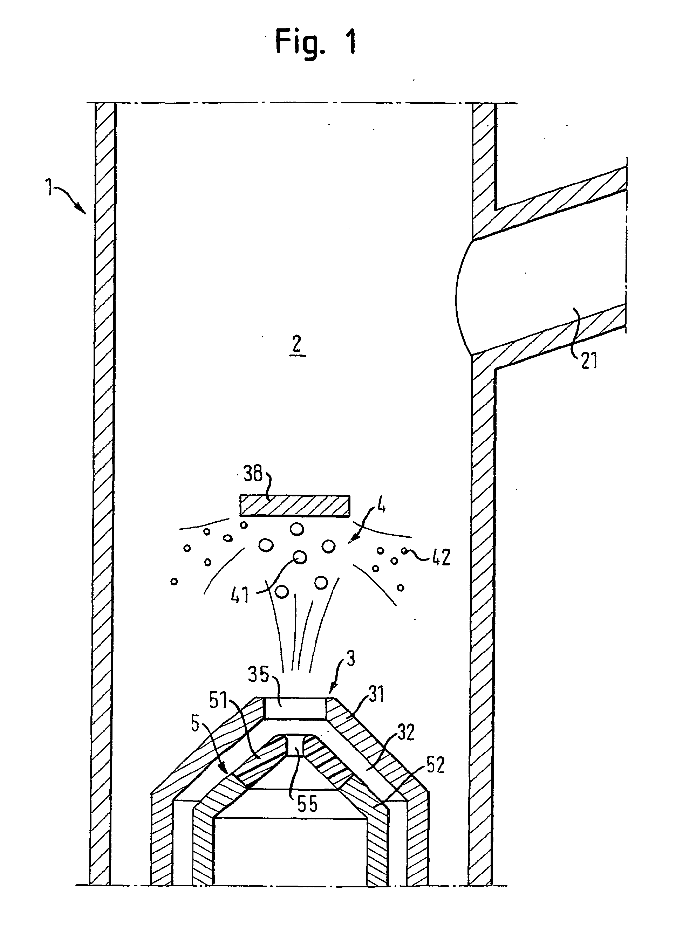

Figur 1 zeigt eine Inhalationstherapievorrichtung 1 gemäß einer ersten Ausführungsform der vorliegenden Erfindung. Die Inhalationstherapievorrichtung umfasst eine Verneblerkammer 2, an die sich zum Beispiel ein Mundstück 21 anschließt, durch das der Patient das vernebelte Medikament in Form eines Aerosols 4 einatmen kann.

Die Inhalationstherapievorrichtung kann weiterhin mit (hier nicht gezeigten) Einatem- und Ausatemventilen versehen sein, so dass ein Atemluftstrom derart geführt werden kann, dass eine optimale Versorgung des Patienten mit einem Aerosol 4 realisiert werden kann. In der Verneblerkammer 2 ist ein Aerosolerzeuger 3 vorgesehen, der in der Lage ist ein Aerosol 4 zu erzeugen.

Der Aerosolerzeuger 3 umfasst ein Düsenelement 5, durch das im vorliegenden Ausführungsbeispiel Druckluft geleitet wird. Weiterhin umfasst der Aerosolerzeuger 3 einen oder mehrere Kanäle 32, durch die ein Medikament aus einem Vorratsbehälter 6 in die Nähe der Düsenöffnung 55 geführt werden kann, aus der die durch das Düsenelement 5 geleitete Druckluft austritt. Die Kanäle können beispielsweise durch ein geeignetes Element 31 gebildet werden, das derart ausgestaltet ist, dass zwischen dem Düsenelement 5 beziehungsweise den Düsenteilen 51, 52 und dem Element 31 ein oder mehrere Kanäle 32 ausgebildet werden. Durch einen Injektions- bzw. Venturi-Effekt wird das Medikament durch die Kanäle 32 angesogen und von der Druckluft, die aus der Düsenöffnung 55 ausströmt, mitgenommen, so dass ein Druckluft-Medikamentengemisch die Öffnung 35 des Elementes 31 durchtritt und in die Verneblerkammer 2 abgegeben wird.

Vor der Öffnung 35 des Aerosolerzeugers 3 befindet sich in diesem Ausführungsbeispiel ein Prallschirm 38, der die Aufgabe hat, den Luftstrom mit dem Medikament derart zu steuern, dass bei einem Aufprall der Medikamententröpfchen auf den Prallschirm 38 ein Aerosol 4, 42 mit einem gewünschten Tröpfchenspektrum entsteht. Die Tröpfchen des aus dem Aerosolerzeuger 3 austretenden Medikamentes 4, 41 prallen auf den Schirm 38, wodurch erreicht wird, dass sich die Tröpfchen des Aerosols teilen und feinere Aerosoltröpfchen 4, 42 bereitgestellt werden können. Durch einen entsprechenden Luftstrom der durch ein eventuell vorhandenes Einatemventil durch von außen zugeführte Atemluft entsteht, wird das Aerosol 4, 42 mitgerissen und von dem Patienten über das Mundstück 21 inhaliert.

Der Düsenkörper 5 des Aerosolerzeugers 3 besteht bei dem hier gezeigten Ausführungsbeispiel aus einem ersten Teil 51 und einem zweiten Teil 52, wobei der erste Teil 51 des Düsenelements 5 einen sich weiter verjüngenden Querschnitt aufweist als der zweite Teil 52 des Düsenelements 5. Der Querschnitt des Düsenkörpers wird durch den ersten Teil des Düsenelements 51 weiter verjüngt, so dass der Querschnitt zur Düsenspitze hin abnimmt. Im hier gezeigten Ausführungsbeispiel umfasst der erste Teil 51 des Düsenelements 5 den Düsenrand beziehungsweise die Düsenkante mit der Düsenaustrittsöffnung 55. Der erste Teil 51 des Düsenelements 5 besteht in diesem Ausführungsbeispiel aus einem elastischeren Material als der zweite Teil 52 des Düsenelements 5.

Unter der Elastizität wird im Sinne der Erfindung eine Materialeigenschaft verstanden, die bewirkt, dass ein aus dem Material gefertigtes Element nach einer Verformung selbständig wieder seine Ursprungsform annimmt. Beispielsweise kann es sich dabei um ein Silikongummi oder ein Elastomer handeln, dass diese Eigenschaft hat, und zwar vorteilhafterweise auch bei einer kerbenden Beanspruchung ohne eine morphologische Schädigung davonzutragen und seine ursprüngliche Form annimmt. Als elastischer wird dabei ein Material bezeichnet, wenn es gegenüber einem weniger elastischen Material in einem höheren Maß wieder die ursprünglichen Form nahezu ohne zurückbleibende Verformung annimmt.

Für die Reinigung des Aerosolerzeugers 3 kann in der Regel das Element 31 abgenommen werden, so dass die Kanäle 32 für das Zuführen des Medikamentes frei liegen, was die Möglichkeit der Reinigung verbessert. Weiterhin wird durch das Abnehmen des Elementes 31 den Düsenkörper derart freigelegt, so dass sie durch eine Spül- beziehungsweise Reinigungsflüssigkeit gereinigt werden kann. Auf Grund der Tatsache, dass der erste Teil 51 des Düsenelements. 5 elastischer ausgebildet ist, als der zweite Teil 52 des Düsenelements 5, kann sich der obere, erste Teil 51 des Düsenelements 5 bei der Reinigung elastisch verformen.

Da es sich bei der elastischen Verformung des Teils 51 des Düsenelements 5 um eine praktisch vollständig reversible Verformung handelt, nimmt der erste Teil 51 des Düsenelements 5 nach der Reinigung wieder die ursprüngliche Form an, so dass die ursprüngliche Geometrie der Düse beibehalten wird, ohne dass Beschädigungen an der Düsengeometrie aufgetreten sind. Der erste Teil 51 des Düsenelements 5 ist an dem zweiten Teil 52 des Düsenelements 5 befestigt bzw. angeformt.

Die mindestens zwei Düsenteile 51, 52 sind wie in dem in Figur 1 gezeigten Ausführungsbeispiel, fest miteinander verbunden, um ein Lösen voneinander zu verhindern und einen reibungs- und turbulenzarmen bzw. -freien Übergang zu gewährleisten.

Fertigungstechnisch steht dafür beispielsweise das so genannte 2-K-Verfahren (Zweikomponentenspritzgussverfahren) zur Verfügung, mit dem möglich ist im Spritzguss zwei oder mehrere geeignete, aber unterschiedliche Materialien, aus denen Teile einer Baueinheit bestehen, in einer Baueinheit einteilig zu fertigen. Die Teile der Baueinheit sind dann fest miteinander verbunden und die Übergänge zueinander können quasi stufen- bzw. absatzfrei und auch im wesentlichen ohne Fuge miteinander verbunden werden. Das gewährleistet in dem vorliegenden Fall einen reibungs- bzw. turbulenzarmen bzw. -freien Übergang.

Der zweite Teil 52 des Düsenelements 5 ist aus einem weniger elastischen Material gefertigt als der erste Teil 51 des Düsenelements 5, so dass dann die Verformbarkeit des ersten Teils ein Nachgeben bei einer mechanischen Reinigung des Düsenelements 5 gewährleistet, wodurch die Beschädigungsgefahr des Düsenelements wesentlich herabgesetzt wird. Das elastische Material kann dabei ein Silikonkautschuk oder ein thermoplastisches Elastomer (TPE) sein. Letzterer lässt sich im Zweikomponentenspritzgussverfahren mit weniger elastischen Materialien, wie etwa Polyethylen (PE) oder Polypropylen (PP), gut verarbeiten, so dass die TPE-Teile und die PE- bzw. PP-Teile fest miteinander verbunden sind.

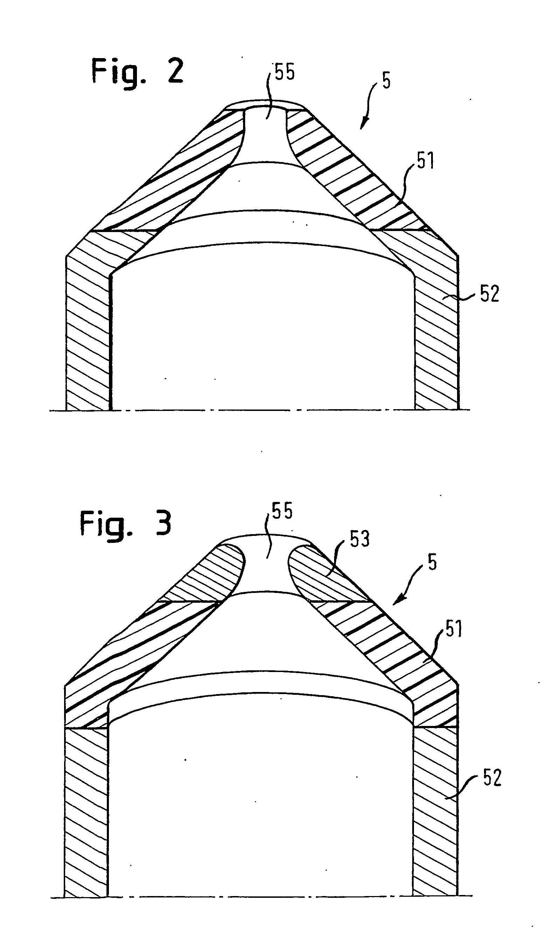

Figur 2 zeigt ein Düsenelement 5, aus einem ersten Teil 51 und einem zweiten Teil 52 besteht. Der erste Teil 51 umfasst die Düsenöffnung 55. Durch den elastischeren Teil 51 des Düsenelements 5 gegenüber dem weniger elastischen Teil 52 des Düsenelements 5 ist die Düsenöffnung 55 beziehungsweise die Düsenkante reversibel verformbar, so dass bei einer Reinigung und einer daraus resultierenden Verformung der Teil 51 des Düsenelements 5 seine ursprüngliche Form wieder annehmen kann, sobald eine mechanische Auswirkung der Reinigung nicht mehr vorliegt.

Vorteilhafterweise kann der Teil 51 des Düsenelements 5 derart ausgestaltet sein, das durch die Wahl eines entsprechenden elastischen Materials die Düsenöffnung 55 in Abhängigkeit von der Druckluftströmung durch die Düsenöffnung 55 aufgeweitet wird, so dass sich ein stabiler Zustand zwischen der durchströmten Luft und dem durch die Kanäle 32 angesaugten Medikament im Aerosolerzeuger 3 einstellen kann. Das ist besonders vorteilhaft, wenn der Druckluft schon in der Zuleitung Partikel beigemengt werden, die gegebenenfalls die Öffnung 55 verstopfen können, so dass durch die angestaute Druckluft die Düsenöffnung aufgeweitet wird, so dass eine Verstopfung der Düsenöffnung vermieden werden kann. Ein weiterer Vorteil eines elastischer ausgestalteten Teils 51, der die Düsenöffnung 55 umfasst, ist die vereinfachte Möglichkeit der Beseitigung fester Verschmutzungspartikel, die fest auf oder in der Düsenspitze anhaften, die bei einer Verformung des elastische Materials abplatzen. Somit wird eine bessere Reinigung gewährleistet. Durch eine elastische Verformung der Düsenöffnung 55 können so auch feste Partikel, die die Düsenöffnung 55 verstopfen entfernt werden, ohne dass die Düsengeometrie nachhaltig geschädigt wird.

Figur 3 zeigt ein Düsenelement 5, das einen dritten Teil 53 umfasst. Der dritte Teil 53 ist wiederum weniger elastisch als der erste Teil 51 des Düsenelements 5. Der dritte Teil 53 des Düsenelements 5 umfasst die Düsenöffnung 55. Der Vorteil ist, dass die Düsenöffnung 55, beziehungsweise der die Düsenöffnung umgebende Rand aus einem formstabileren Material gefertigt sein kann, jedoch auf Grund des elastischeren Teils 51 auf Druck, beziehungsweise auf Grund anderer mechanischer Einwirkungen derart nachgeben kann, so dass keine Beschädigung an dem oberen Teil, hier dem dritten Teil 53 des Düsenelements 5 auftreten können. Bei einer Reinigung der Düse verformt sich in der elastische Teile 51 des Düsenelements 5 und nimmt, nachdem keine mechanische Einwirkung mehr besteht, die ursprüngliche Form wieder an, so dass die Formstabilität der Düse gewährleistet bleibt.

Die Anforderungen an die Handhabbarkeit, die mechanischen Auswirkungen, die auszuhalten sind, und die zu erwartenden Beanspruchungen bestimmen die Auswahl des Materials für die jeweiligen Teile 51, 52, 53, wobei davon ausgegangen wird, dass der Fachmann geeignete Materialien mit geeigneten Elastizitäten auswählt.

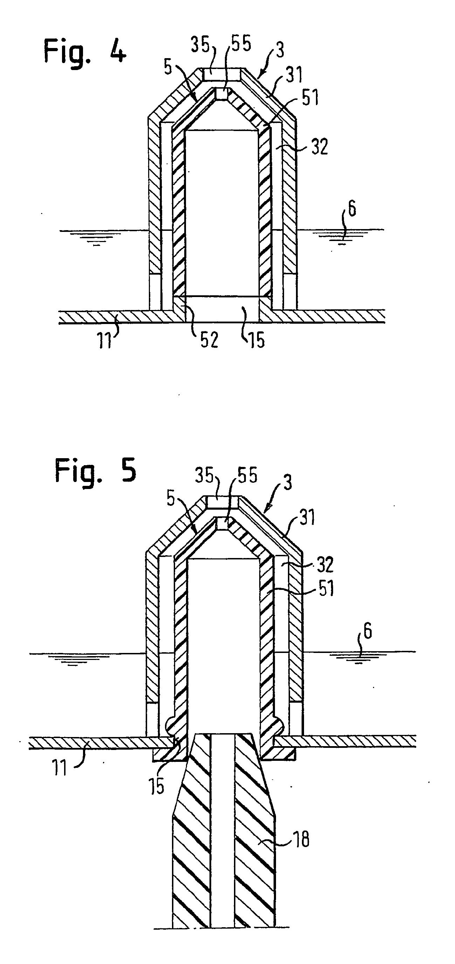

Wie Figur 4 zeigt, muss die Grenze zwischen dem ersten Teil 51 des Düsenelements 5 und dem zweiten Teil des Düsenelementes nicht zwangsweise im oberen Bereich des Düsenelements 5 liegen. Vielmehr kann der Verbindungsbereich zwischen dem ersten Teil 51 des Düsenelements 5 und dem zweiten Teil 52 des Düsenelements 5 auch im unteren Bereich des Düsenelementes liegen, der beispielsweise an einen Gehäuseteil 11 anschließt.

Gemäß eines weiteren Ausführungsbeispiels kann das Düsenelement auch vollständig aus einem elastischeren Material gefertigt sein als ein Bauteil 11 der Inhalationstherapievorrichtung, an das das Düsenelement 5 angeformt ist, bzw. an dem das Düsenelement befestigt ist. Das Düsenelement kann, wie in Figur 5 gezeigt ist, beispielsweise als steckbares Element ausgeführt sein, das bei der Fertigung in eine vorgesehene Öffnung 15 eingesteckt wird. Dadurch wird die Fertigung vereinfacht und das Düsenelement 5 kann gegebenenfalls bei eine Beschädigung ausgetauscht werden. Ferner kann das Düsenelement 5 Dichtungsfunktionen wahrnehmen, beispielsweise bei einer angesetzten Schlauchzuführung 18.

Das Düsenelement 5 kann,dabei vorteilhafterweise aus einem elastischen Material wie etwa Silikonkautschuk oder einem thermoplastischen Elastomer (TPE) sein. Durch das Element 31 wird die Formstabilität im Betrieb gewährleistet. Beim Abnehmen des Elementes 31 liegt das Düsenelement 5 frei und ist aufgrund seiner Elastizität bei einer Reinigung nicht in dem Maße einer Beschädigungsgefahr ausgesetzt.

## Patentansprüche

1. Inhalationstherapievorrichtung mit:
- einer Verneblerkammer (2), und
- einem Aerosolerzeuger (3), der derart angeordnet ist, dass er ein Aerosol (4) in die Verneblerkammer (2) abgeben kann, und der ein Düsenelement (5) umfasst,
wobei das Düsenelement (5) aus wenigstens einem ersten Teil (51) und einem zweiten Teil (52) besteht, wobei der erste Teil (51) des Düsenelements (5) an dem zweiten Teil (52) des Düsenelements (5) befestigt ist, **dadurch gekennzeichnet, dass** der erste Teil (51) des Düsenelements (5) aus einem elastischeren Material besteht als der zweite Teil (52) des Düsenelements (5).

2. Inhalationstherapievorrichtung nach Anspruch 1, **dadurch gekennzeichnet, dass** der erste Teil (51) des Düsenelements (5) einen sich weiter verjüngenden Querschnitt aufweist als der zweite TeiL (52) des Düsenelements (5).

3. Inhalationstherapievorrichtung nach einem der vorangegangenen Ansprüche, **dadurch gekennzeichnet, dass** der erste Teil (51) des Düsenelements (5) aus Silikongummi oder einem thermoplastischen Elastomer besteht.

4. Inhalationstherapievorrichtung nach einem der vorangegangenen Ansprüche, **dadurch gekennzeichnet, dass** der erste Teil (51) des Düsenelements (5) mit dem zweiten Teil (52) des Düsenelements (5) im 2-K-Verfahren gefertigt ist und **dadurch** der erste Teil (51) des Düsenelements (5) an den zweiten Teil (52) des Düsenelements (5) angeformt ist.

5. Inhalationstherapievorrichtung nach einem der vorangegangenen Ansprüche, **dadurch gekennzeichnet, dass** der erste Teil (51) des Düsenelements (5) die Düsenaustrittsöffnung (55) umfasst .

6. Inhalationstherapievorrichtung nach einem der vorangegangenen Ansprüche 1 bis 4, **dadurch gekennzeichnet, dass** die Düse (5) einen dritten Teil (53) umfasst, der die Düsenaustrittsöffnung (55) umfasst.

7. Inhalationstherapievorrichtung nach Anspruch 6, **dadurch gekennzeichnet, dass** der dritte Teil (53) des Düsenelements (5) einen sich weiter verjüngenden Querschnitt aufweist, als der erste Teil (51) des Düsenelements (5).

8. Inhalationstherapievorrichtung nach einem der Ansprüche 6 oder 7, **dadurch gekennzeichnet, dass** der dritte Teil (53) des Düsenelements (5) mit dem ersten Teil (51)des Düsenelements (5) im 2-K-Verfahren hergestellt wird.

9. Inhalationstherapievorrichtung nach einem der Ansprüche 6 bis 8, **dadurch gekennzeichnet, dass** der dritte Teil (53) des Düsenelements (5) aus einem weniger elastischen Material als der erste Teil (51) des Düsenelements (5) besteht.

10. Inhalationstherapievorrichtung mit:
- einer Verneblerkammer (2), und
- einem Aerosolerzeuger (3), der derart angeordnet ist, dass er ein Aerosol (4) in die Verneblerkammer (2) abgeben kann und der ein Düsenelement (5) umfasst,
wobei das Düsenelement (5) aus wenigstens einem ersten Teil (51) besteht, **dadurch gekennzeichnet, dass** der erste Teil (5i) des Düsenelements (5) aus einem elastischeren Material besteht als ein Element (11) der Inhalationstherapievorrichtung (1), an den das Düsenelement (5) angeformt ist, beziehungsweise an dem das Düsenelement (5) befestigt ist.

11. Inhalationstherapievorrichtung nach Anspruch 10, **dadurch gekennzeichnet, dass** der erste Teil (51) des Düsenelements (5) aus Silikongummi oder einem thermoplastischen Elastomer besteht.

12. Inhalationstherapievorrichtung nach Anspruch 10 oder 11, **dadurch gekennzeichnet, dass** der erste Teil (51) des Düsenelements (5) die Düsenaustrittsöffnung (55) umfasst.

## Claims

1. An Inhalation therapy device comprising:
- a nebulising chamber (2), and
- an aerosol generator (3), which is arranged such that it can release an aerosol (4) into the nebulising chamber (2), and which comprises a nozzle element (5),
said nozzle element (5) consisting of at least a first part (51) and a second part (52), said first part (51) of the nozzle element (5) being attached to said second part (52) of the nozzle element (5), **characterized in that**
said first part (51) of the nozzle element (5) being made of a more resilient material than said second part (52) of the nozzle element (5).

2. An inhalation therapy device according to claim 1, **characterised in that** the first part (51) of the nozzle element (5) has a cross-section which tapers further than that of the second part (52) of said nozzle element (5).

3. An inhalation therapy device according to one of the preceding claims, **characterised in that** the first part (51) of the nozzle element (5) is made of silicone rubber or a thermoplastic elastomer.

4. An inhalation therapy device according to one of the preceding claims, **characterised in that** the first part (51) of the nozzle element (5) is produced together with the second part (52) of said nozzle element (5) in a two-component method and said first part (51) of the nozzle element (5) is thereby moulded on said second part (52) of the nozzle element (5).

5. An inhalation therapy device according to one of the preceding claims, **characterised in that** the first part (51) of the nozzle element (5) contains the nozzle outlet (55).

6. An inhalation therapy device according to one of the preceding claims 1 to 4, **characterised in that** the nozzle (5) has a third part (53) containing the nozzle outlet (55).

7. An inhalation therapy device according to claim 6, **characterised in that** the third part (53) of the nozzle element (5) has a cross-section which tapers further than that of the first part (51) of the nozzle element (5).

8. An inhalation therapy device according to one of claims 6 or 7, **characterised in that** the third part (53) of the nozzle element (5) is produced together with the first part (51) of the nozzle element (5) in the two-component method.

9. An inhalation therapy device according to one of claims 6 to 8, **characterised in that** the third part (53) of the nozzle element (5) is made of a less resilient material than the first part (51) of said nozzle element (5).

10. An inhalation therapy device comprising:
- a nebulising chamber (2), and
- an aerosol generator (3), which is arranged such that it can release an aerosol (4) into the nebulising chamber (2), and which comprises a nozzle element (5),
said nozzle element (5) consisting of at least a first part (51), **characterized in that** said first part (51) of the nozzle element (5) being made of a more resilient material than a member (11) of the inhalation therapy device (1) on which the nozzle element (5) is moulded or to which the nozzle element (5) is attached.

11. An inhalation therapy device according to claim 10, **characterised in that** the first part (51) of the nozzle element (5) is made of silicone rubber or a thermoplastic elastomer.

12. An inhalation therapy device according to claim 10 or 11, **characterised in that** the first part (51) of the nozzle element (5) contains the nozzle outlet (55).

## Revendications

1. Dispositif de traitement par inhalation, comprenant :
- une chambre de nébulisateur (2), et
- un générateur d'aérosol (3), disposé de manière qu'il puisse délivrer un aérosol (4) dans la chambre de nébulisateur (2), et comprenant un élément formant buse (5),
l'élément formant buse (5) étant composé d'au moins une première partie (51) et d'une deuxième partie (52), la première partie (51) de l'élément formant buse (5) étant fixée sur la deuxième partie (52) de l'élément formant buse (5), **caractérisé en ce que** la première partie (51) de l'élément formant buse (5) est composée d'un matériau plus élastique que la deuxième partie (52) de l'élément formant buse (5) .

2. Dispositif de traitement par inhalation selon la revendication 1, **caractérisé en ce que** la première partie (51) de l'élément formant buse (5) présente une section transversale allant en s'effilant plus loin que la deuxième partie (52) de l'élément formant buse (5).

3. Dispositif de traitement par inhalation selon l'une des revendications précédentes, **caractérisé en ce que** la première partie (51) de l'élément formant buse (5) est composée de caoutchouc au silicone, ou d'un élastomère thermoplastique.

4. Dispositif de traitement par inhalation selon l'une des revendications précédentes, **caractérisé en ce que** la première partie (51) de l'élément formant buse (5) est fabriquée avec la deuxième partie (52) de l'élément formant buse (5) dans un procédé de type 2-K (procédé de moulage par injection à deux composants) et de ce fait, la première partie (51) de l'élément formant buse (5) est formée d'un seul tenant sur la deuxième partie (52) de l'élément formant buse (5).

5. Dispositif de traitement par inhalation selon l'une des revendications précédentes, **caractérisé en ce que** la première partie (51) de l'élément formant buse (5) comprend l'ouverture de sortie de buse (55).

6. Dispositif de traitement par inhalation selon l'une des revendications 1 à 4 précédentes, **caractérisé en ce que** la buse (5) comprend une troisième partie (53), qui comprend l'ouverture de sortie de buse (55).

7. Dispositif de traitement par inhalation selon la revendication 6, **caractérisé en ce que** la troisième partie (53) de l'élément formant buse (5) présente une section transversale s'effilant plus loin que la première partie (51) de l'élément formant buse (5).

8. Dispositif de traitement par inhalation selon l'une des revendications 6 ou 7, **caractérisé en ce que** la troisième partie (53) de l'élément formant buse (5) est fabriquée avec la première partie (51) de l'élément formant buse (5), dans un procédé 2-K.

9. Dispositif de traitement par inhalation selon l'une des revendications 6 à 8, **caractérisé en ce que** la troisième partie (53) de l'élément formant buse (5) est composée d'un matériau moins élastique que la première partie (51) de l'élément formant buse (5).

10. Dispositif de traitement par inhalation, comprenant :
- une chambre de nébulisateur (2), et
- un générateur d'aérosol (3), disposé de manière qu'il puisse délivrer un aérosol (4) dans la chambre de nébulisateur (2), et comprenant un élément formant buse (5),
sachant que l'élément formant buse (5) est composé d'au moins une première partie (51), **caractérisé en ce que** la première partie (51) de l'élément formant buse (5) est composée d'un matériau plus élastique qu'un élément (11) du dispositif de traitement par inhalation (1), sur lequel l'élément formant buse (5) est formé d'un seul tenant, ou est fixé sur l'élément formant buse (5).

11. Dispositif de traitement par inhalation selon la revendication 10, **caractérisé en ce que** la première partie (51) de l'élément formant buse (5) est composée de caoutchouc au silicone, ou d'un élastomère thermoplastique.

12. Dispositif de traitement par inhalation selon la revendication 10 ou 11, **caractérisé en ce que** la première partie (51) de l'élément formant buse (5) entoure l'ouverture de sortie de buse (55).
